# EUROPEAN PATENT SPECIFICATION

(11) **EP 0 592 790 B1**
(45) Date of publication and mention of the grant of the patent: **11.03.1998**
(21) Application number: 93113271.6
(22) Date of filing: 19.08.1993
(51) Int. Cl.: G03F 7/004, G03F 7/029, G03F 7/038

(54) **Negative photoresist composition**
Negativ arbeitende Photoresistzusammensetzung
Composition photorésistante négative

(30) Priority: 20.08.1992 JP 221374/92
(43) Date of publication of application: 20.04.1994
(73) Proprietor: SUMITOMO CHEMICAL COMPANY LIMITED, Osaka-shi, Osaka 541 (JP)
(72) Inventor: Ueda, Yuji, Izumi-shi (JP); Takeyama, Naoki, Settsu-shi (JP); Ueki, Hiromi, Mishima-gun, Osaka (JP); Kusumoto, Takehiro, Takarazuka-shi (JP); Nakano, Yuko, Ibaraki-shi (JP)
(74) Representative: VOSSIUS & PARTNER

(56) References cited:
- EP-A- 0 407 086
- EP-A- 0 483 693
- US-A- 4 189 323
- PATENT ABSTRACTS OF JAPAN vol. 016, no. 407 (P-1411)27 August 1992 & JP-A-41 36 858 ( TOKYO OHKA KOGYO CO., LTD. ) 11 May 1992
- PATENT ABSTRACTS OF JAPAN vol. 016, no. 407 (P-1411)27 August 1992 & JP-A-41 36 859 ( TOKYO OHKA KOGYO CO., LTD. ) 11 May 1992

## Description

The present invention relates to a resist composition usable as a pattern-forming material in the production of semiconductors, and more particularly to a negative photoresist composition suitable for use in lithographies using far ultraviolet rays including excimer laser.

Recently, with a rise in the integration level of integrated circuits, formation of patterns of sub-micron order is required. Particularly, excimer laser lithography using excimer laser as a light source makes it possible to produce 64 and 256 MDRAMs. As a result of such an alteration of light source, the following properties are demanded of the today's resists in addition to the hitherto required properties such as heat resistance, or film thickness retention:
(1) a high sensitivity to the light sources mentioned above,
(2) a high resolution,
(3) a good profile, and
(4) a small standing wave.

Prior negative photoresist compositions cannot be said to be satisfactory from the above-mentioned viewpoints. For example, JP-A-1-293339 (1989) discloses a photoresist composition comprising a polymer containing a group represented by the following formula (I): 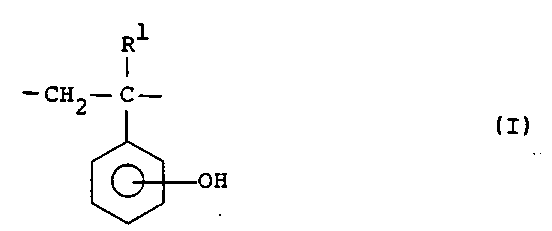 wherein R¹ represents a hydrogen atom or an alkyl group having from 1 to 5 carbons atoms, a crosslinking agent containing a group represented by the following formula: 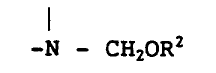 wherein R² represents a hydrogen atom or an alkyl group having 1 to 5 carbon atoms, and a photo-induced acid precursor containing a sulfonic acid ester group. If a pattern is formed from this composition, however, the profile is not good or the film thickness retention is low and a pattern of 0.4 µm or less cannot sufficiently be resolved. Thus, it is the object of the present invention to solve the problems mentioned above and to provide a negative photoresist composition suitable for use in far ultraviolet lithographies and particularly KrF excimer laser lithography, excellent in properties such as resolution, sensitivity, heat resistance, profile or film thickness retention, and small in standing wave. This object has been achieved by a negative photoresist composition comprising 20 to 90 % by weight of a copolymer of p-hydroxystyrene and styrene having a molar ratio of p-hydroxystyrene to styrene of from 50/50 to 95/5, 5 to 50% by weight of a crosslinking agent represented by the following general formula (II): 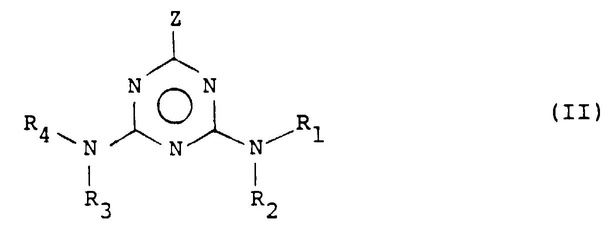 wherein Z represents -NRR' or an optionally substituted aryl group, and R, R' and R₁ to R₄ independently of one another each represents a hydrogen atom, a -CH₂OH group or a -CH₂ORₐ group in which Rₐ is a lower alkyl group, and 0.1 to 20% by weight of a photo-induced acid precursor represented by the following general formula (III): 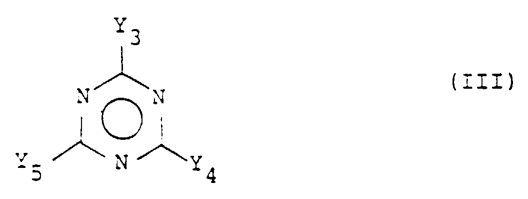 wherein Y₃, Y₄, and Y₅ independently of one another each represents an optionally substituted alkyl, alkenyl, aralkyl or piperidino group, a -N(Y₆)(Y₇) group, an -OY₈ group or a -SY₉ group in which Y₆ to Y₉ independently of one another each represents a hydrogen atom or an optionally substituted alkyi or aryl group, provided that at least one of Y₃, Y₄ and Y₅ represents mono-, di- or tri-halogen-substituted alkyl group, or 2-phenyl-4,6-bis (trichloromethyl)-s-triazine, 2-(p-acethylphenyl)-4,6-bis(trichloromethyl-s-triazine or 2-(p-chlorophenyl)4,6-bis(trichloromethyl)-s-triazine.

The above-mentioned resin can easily be produced by a radical copolymerization or ion copolymerization of p-hydroxystyrene and styrene. The molar ratio of p-hydroxystyrene and styrene in the copolymer is from about 50/50 to about 95/5, and preferably from about 70/30 to about 85/15. If the proportion of p-hydroxystyrene is smaller than the above, the copolymer is difficult to dissolve in solvent at the time of preparing a resist solution or difficult to dissolve in aqueous alkali solution at the time of development. On the other hand, if the proportion of p-hydroxystyrene is greater than the above, the copolymer is too readily soluble in aqueous alkali solution, so that no fine pattern can be formed therefrom. A weight average molecular weight of the resin is usually from about 1,000 to about 5,000, preferably from about 2,500 to about 4,000.

In the crosslinking agent represented by the general formula (II), preferable examples of the lower alkyl group represented by Rₐ include straight chain and branched chain alkyl groups having 1 to 7 carbon atoms among which methyl, ethyl, n-propyl and n-butyl are preferable and methyl and ethyl are more preferable, and examples of the optionally substituted aryl group include optionally substituted phenyl or naphtyl. Among the crosslinking agents represented by the general formula (II), melamine and benzoguanamine are readily available commercially, and their methylol derivatives are obtainable through a condensation reaction of melamine or benzoguanamine with formalin. Their methylol ether derivatives can be obtained by reacting a methylol derivative with an alcohol according to known methods.

In the general formula (III), examples of the alkyl group represented by Y₃ to Y₅ include straight chain and branched chain alkyl groups having 1 to 7 carbon atoms, among which methyl, ethyl, n-propyl, i-propyl, n-butyl and t-butyl are preferable. As the substituent present on the alkyl group, halogen atoms

can be referred to, for example. Preferable examples of the alkenyl group represented by Y₃ to Y₅ include straight chain and branched chain alkenyl groups having 2 to 7 carbon atoms. As the substituent present on the alkenyl group, aryl groups such as phenyl or naphthyl and heterocyclic groups such as benzoxazolyl or benzothiazolyl can be referred to, for example. These aryl groups and heterocyclic groups may be additionally substituted by halogen atoms, alkoxy groups having 1 to 7 carbon atoms, an acetyl group or a nitro group.

Preferable examples of the aralkyl group represented by Y₃ to Y₅ include phenylalkyl groups having 7 to 10 carbon atoms. As preferable substituents present on the phenyl moiety of these aralkyl groups, lower alkyl groups, lower alkoxy groups, halogen atom, nitro group, cyano group, dimethylamino group and diethylamino group can be referred to. Preferable examples of the optionally substituted alkyl group represented by Y₆ to Y₉ include straight chain and branched chain alkyl groups having 1 to 6 carbon atoms, among which methyl, ethyl, n-propyl, and n-butyl are more preferable. Preferable examples of the optionally substituted aryl group represented by Y₆ to Y₉ include a phenyl or naphthyl group among which the phenyl group is more preferable.

The photo-induced acid precursor represented by the formula (III) can be produced by, for example, reacting the corresponding nitrile compound with aluminum bromide in the presence of hydrogen chloride or by using the corresponding imidate compound, according to Bull. Chem. Soc. Japan, 42, 2924 (1969); US-A- 3,987,037; or F. C. Schaefer et. al, J. Org. Chem. 29, 1527 (1964). Examples of the photo-induced acid precursor represented by the formula (III) include the compound disclosed in the papers mentioned above and the compound disclosed in GB-B- 1,388,492 and JP-A-53-133428 (1978).

Among these compounds, preferable are 2,4,6-tris(trichloromethyl)-s-triazine, 2-phenyl-4,6-bis(trichloromethyl)-s-triazine, 2,4,6-tris(dibromopropyl)-s-triazine, 2-(p-acetylphenyl)-4,6-bis(trichloromethyl)-s-triazine, 2-(p-chlorophenyl)-4,6-bis(trichloromethyl)-s-triazine and 2-(p-methoxyphenyl)-4,6-bis(trichloromethyl)-s-triazine. These photo-induced acid precursors may be used either singly or in the form of a mixture of two or more members.

In the negative photoresist composition of the present invention, the above-mentioned resin is used usually in an amount of from 20 to 90% by weight (preferably in an amount of from 40 to 80% by weight), the crosslinking agent is used usually in an amount of from 5 to 50% by weight (preferably in an amount of from 5 to 20% by weight) and the photo-induced acid precursor is used usually in an amount of from 0.1 to 20% by weight (preferably in an amount of from 1 to 20% by weight), respectively. If necessary, the negative photoresist composition of the present invention may contain a sensitizer, and a dye.

Generally speaking, a negative resist solution is prepared by mixing the above-mentioned resin, a crosslinking agent, and a photo-induced acid precursor into a solvent. These ingredients are used in an amount of from 1 to 50% by weight in the resulting negative resist solution. The solvent is preferably one that evaporates at an appropriate drying rate to give a uniform and smooth coating film. Examples of such solvent include methyl cellosolve acetate, ethyl cellosolve acetate, methyl cellosolve, ethyl cellosolve, propylene glycol monomethyl ether acetate, propylene glycol monoethyl ether acetate, butyl acetate, methyl isobutyl ketone, xylene, ethyl lactate, ethyl pyruvate and 2-heptanone. These solvents are used either singly or in the form of a mixture of two or more members.

Next, the present invention will be explained by the following examples.

In examples, "parts" are by weight.

### Example 1

50 Parts of diethylene glycol dimethyl ether were mixed with 13.33 parts of a resin [LYNCUR CST-70, manufactured by Maruzen Sekiyukagaku K. K., weight average molecular weight 2,900, p-hydroxystyrene/styrene molar ratio in copolymer 70/30], 1 part of hexamethylolmelamine hexamethyl ether as a crosslinking agent and 3 parts of tris(2,3-dibromopropyl)-1,3,5-triazine (a reagent manufactured by Tokyo Kasei Kogyo K. K.] as a photo-induced acid precursor. The resulting mixture was filtered through a tetrafluoroethylene filter having a pore size of 0.2 µm to obtain a resist solution. Using a spinner, the resist solution was coated on a silicon wafer which had been rinsed in a usual manner to form a resist film of 0.7 µm in thickness. Subsequently, the silicon wafer was pre-baked in an oven at 100°C for one minute, and the coated film on the wafer was exposed to light by means of KrF Excimer Laser Stepper (manufactured by Nikon Co., NSR1755EX NA=0.45) having an exposure wavelength of 248 nm. After the exposure, the wafer was heated on a hot plate at 100°C for one minute to make progress a crosslinking reaction of the exposed area. Then, the wafer was developed with a 2.0% (by weight) aqueous solution of tetramethylammonium hydroxide to obtain a negative pattern. An observation by means of an electron microscope revealed that a 0.25 µm fine pattern was resolved with a good profile, provided that exposure dose was 60 mJ/cm² (effective sensitivity).

### Example 2

A negative pattern was obtained by repeating Example 1, except that the resin was altered to LYNCUR CST-50 (manufactured by Maruzen Sekiyukagaku K. K., weight average molecular weight 3,600, p-hydroxystyrene/styrene ratio in copolymer 50/50). A 0.25 µm fine pattern was resolved with a good profile, provided that exposure dose was 80 mJ/cm² (effective sensitivity).

### Comparative Example 1

The procedure of Example 1 was repeated, except that the resin was altered to 13.33 parts of poly(p-hydroxystyrene) (MARUKA LYNCUR M, manufactured by Maruzen Sekiyukagaku K. K., weight average molecular weight 4,100). As a result, a sensitivity of 35 mJ/cm² was obtained. However, a 0.25 µm pattern could not be resolved.

## Claims

1. (Amended) A negative photoresist composition comprising 20 to 90% by weight of a copolymer of p-hydroxystyrene and styrene having a molar ratio of p-hydroxystyrene to styrene of from 50/50 to 95/5, 5 to 50% by weight of a crosslinking agent represented by the formula (II): 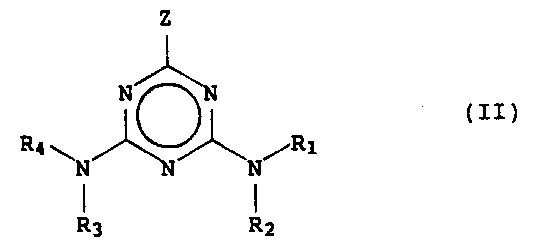 wherein Z represents -NRR' or an optionally substituted aryl group, and R, R', R₁, R₂, R₃ and R₄ independently of one another each represents a hydrogen atom, a -CH₂OH group or a -CH₂ORₐ group in which Rₐ is a lower alkyl group, and
0.1 to 20% by weight of a photo-induced acid precursor represented by the formula (III): 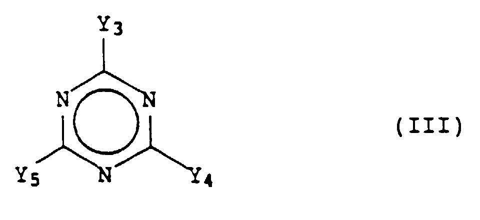 wherein Y₃, Y₄ and Y₅ independently of one another each represents an optionally substituted alkyl, alkenyl, aralkyl or piperidino group, a -N(Y₆)(Y₇) group, an -OY₈ group or a -SY₉ group in which Y₆, Y₇, Y₈ and Y₉ independently of one another each represents a hydrogen atom or an optionally substituted alkyl or aryl group, provided that at least one of Y₃, Y₄ and Y₅ represents a mono-, di- or tri-halogen-substituted alkyl group, or 2-phenyl-4,6-bis(trichloromethyl)-s-triazine, 2-(p-acetylphenyl)-4,6 -bis(trichloromethyl)-s-triazine or 2-(p-chlorophenyl)4,6-bis(trichloromethyl)-s-triazine.

2. The negative photoresist composition according to claim 1, wherein the molar ratio of p-hydroxystyrene to styrene in the copolymer is from 70/30 to 85/15.

3. The negative photoresist composition according to claim 1, wherein the weight average molecular weight of the copolymer is from 1,000 to 5,000.

4. The negative photoresist composition according to claim 1, wherein the weight average molecular weight of the copolymer is from 2,500 to 4,000.

5. The negative photoresist composition according to claim 1, wherein the lower alkyl group represented by Rₐ is selected from the group consisting of methyl, ethyl, n-propyl and n-butyl.

6. The negative photoresist composition according to claim 1, wherein the crosslinking agent is melamine or benzoguanamine.

7. The negative photoresist composition according to claim 1, wherein the alkyl group represented by Y₃, Y₄ and Y₅ is a straight or branched chain alkyl group having 1 to 7 carbon atoms that may be substituted with a halogen atom.

8. The negative photoresist composition according to claim 1, wherein the alkyl group represented by Y₃, Y₄ and Y₅ is selected from the group consisting of methyl, ethyl, n-propyl, i-propyl, n-butyl and t-butyl groups that may be substituted with a halogen atom.

9. The negative photoresist composition according to claim 1, wherein the alkenyl group represented by Y₃, Y₄ and Y₅ is a straight or branched chain alkenyl group having 2 to 7 carbon-atoms that may be substituted with an aryl group or heterocyclic group.

10. The negative photoresist composition according to claim 1, wherein the aralkyl group represented by Y₃, Y₄ and Y₅ is a phenylalkyl group having 7 to 10 carbon atoms.

11. The negative photoresist composition according to claim 1, wherein the optionally substituted alkyl group represented by Y₆ to Y₉ is a straight or branched chain alkyl group having 1 to 6 carbon atoms.

12. The negative photoresist composition according to claim 11, wherein the optionally substituted alkyl group represented by Y₆ to Y₉ is selected from the group consisting of methyl, ethyl, n-propyl and n-butyl groups.

13. The negative photoresist composition according to claim 1, wherein the optionally substituted aryl group represented by Y₆ to Y₉ is a phenyl or naphthyl group.

14. The negative photoresist composition according to claim 1, wherein the composition comprises 40 to 80% by weight of the copolymer, 5 to 20% by weight of the crosslinking agent and 1 to 20% by weight of the photo-induced acid precursor.

## Patentansprüche

1. Negativ arbeitende Photoresistzusammensetzung, umfassend 20 bis 90 Gewichts-% eines Copolymers von p-Hydroxystyrol und Styrol mit einem Molverhältnis von p-Hydroxystyrol zu Styrol von 50/50 bis 95/5, 5 bis 50 Gewichts-% eines Vernetzungsmittels der Formel (II): 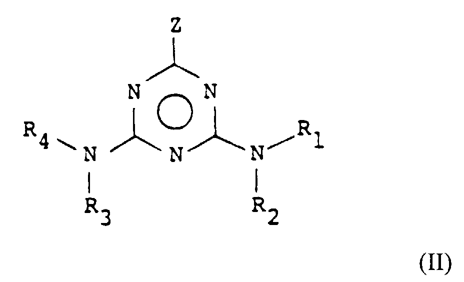 in der Z den Rest -NRR' oder einen gegebenenfalls substituierten Arylrest bedeutet, und R, R', R₁, R₂, R₃ und R₄ jeweils unabhängig voneinander ein Wasserstoffatom, eine -CH₂OH-Gruppe oder einen -CH₂ORₐ-Rest bedeuten, wobei Rₐ ein Niederalkylrest ist, und
0,1 bis 20 Gewichts-% einer licht-induzierten Säurevorstufe der Formel (III): 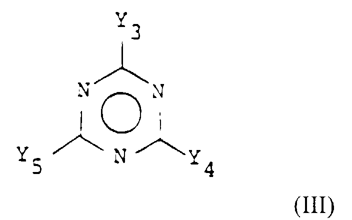 in der Y₃, Y₄ und Y₅ jeweils unabhängig voneinander einen gegebenenfalls substituierten Alkyl-, Alkenyl-, Aralkyl- oder Piperidinorest, einen -N(Y₆)(Y₇)-Rest, einen -OY₈-Rest oder einen -SY₉-Rest bedeuten, wobei Y₆, Y₇, Y₈ und Y₉ jeweils unabhängig voneinander ein Wasserstoffatom oder einen gegebenenfalls substituierten Alkyl- oder Arylrest bedeuten, mit der Maßgabe, daß wenigstens einer der Reste Y₃, Y₄ und Y₅ einen mono-, di- oder trihalogensubstituierten Alkylrest darstellt,
oder 2-Phenyl-4,6-bis(trichlormethyl)-s-triazin, 2-(p-Acetylphenyl)-4,6-bis(trichlormethyl)-s-triazin oder 2-(p-Chlorphenyl)-4,6-bis(trichlormethyl)-s-triazin.

2. Negativ arbeitende Photoresistzusammensetzung nach Anspruch 1, wobei das Molverhältnis von p-Hydroxystyrol zu Styrol im Copolymer von 70/30 bis 85/15 beträgt.

3. Negativ arbeitende Photoresistzusammensetzung nach Anspruch 1, wobei das gewichtsgemittelte Molekulargewicht des Copolymers von 1.000 bis 5.000 beträgt.

4. Negativ arbeitende Photoresistzusammensetzung nach Anspruch 1, wobei das gewichtsgemittelte Molekulargewicht des Copolymers von 2.500 bis 4.000 beträgt.

5. Negativ arbeitende Photoresistzusammensetzung nach Anspruch 1, wobei der durch Rₐ wiedergegebene Niederalkylrest aus der Methyl-, Ethyl-, n-Propyl- und n-Butylgruppe ausgewählt wird.

6. Negativ arbeitende Photoresistzusammensetzung nach Anspruch 1, wobei das Vernetzungsmittel Melamin oder Benzoguanamin ist.

7. Negativ arbeitende Photoresistzusammensetzung nach Anspruch 1, wobei der durch Y₃, Y₄ und Y₅ wiedergegebene Alkylrest ein gerad- oder verzweigtkettiger Alkylrest mit 1 bis 7 Kohlenstoffatomen ist, der mit einem Halogenatom substituiert sein kann.

8. Negativ arbeitende Photoresistzusammensetzung nach Anspruch 1, wobei der durch Y₃, Y₄ und Y₅ wiedergegebene Alkylrest aus Methyl-, Ethyl, n-Propyl-, 1-Propyl-, n-Butyl und t-Butylgruppen, die mit einem Halogenatom substituiert sein können, ausgewählt wird.

9. Negativ arbeitende Photoresistzusammensetzung nach Anspruch 1, wobei der durch Y_{3,} Y₄ und Y₅ wiedergegebene Alkenylrest ein gerad- oder verzweigtkettiger Alkenylrest mit 2 bis 7 Kohlenstoffatomen ist, der mit einem Arylrest oder einem heterocyclischen Rest substituiert sein kann.

10. Negativ arbeitende Photoresistzusammensetzung nach Anspruch 1, wobei der durch Y₃, Y₄ und Y₅ wiedergegebene Aralkylrest ein Phenylalkylrest mit 7 bis 10 Kohlenstoffatomen ist.

11. Negativ arbeitende Photoresistzusammensetzung nach Anspruch 1, wobei der gegebenenfalls substituierte, durch Y6 bis Y₉ wiedergegebene Alkylrest ein gerad- oder verzweigtkettiger Alkylrest mit 1 bis 6 Kohlenstoffatomen ist

12. Negativ arbeitende Photoresistzusammensetzung nach Anspruch 11, wobei der gegebenenfalls substituierte, durch Y₆ bis Y₉ wiedergegebene Alkylrest aus Methyl-, Ethyl-, n-Propyl- und n-Butylgruppen ausgewählt wird.

13. Negativ arbeitende Photoresistzusammensetzung nach Anspruch 1, wobei der gegebenenfalls substituierte, durch Y₆ bis Y₉ wiedergegebene Arylrest eine Phenyl- oder Naphthylgruppe ist.

14. Negativ arbeitende Photoresistzusammensetzung nach Anspruch 1, wobei die Zusammensetzung 40 bis 80 Gewichts-% des Copolymers, 5 bis 20 Gewichts-% des Vernetzungsmittels und 1 bis 20 Gewichts-% der lichtinduzierten Säurevorstufe umfaßt.

## Revendications

1. Composition de photoresist négatif comprenant 20 à 90 % en poids d'un copolymère de p-hydroxystyrène et de styrène ayant un rapport molaire du p-hydroxystyrène au styrène de 50/50 à 95/5, 5 à 50 % en poids d'un agent de réticulation représenté par la formule (II) : 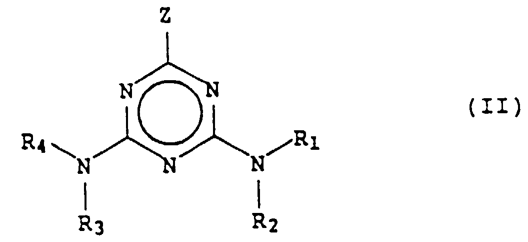 dans laquelle Z représente -NRR' ou un groupe aryle éventuellement substitué, et R, R', R₁, R₂, R₃ et R₄, indépendamment les uns des autres, représentent chacun un atome d'hydrogène, un groupe -CH₂OH ou un groupe -CH₂ORₐ dans lequel Rₐ est un groupe alkyle inférieur, et
0,1 à 20 % en poids d'un précurseur d'acide photo-induit représenté par la formule (III) : 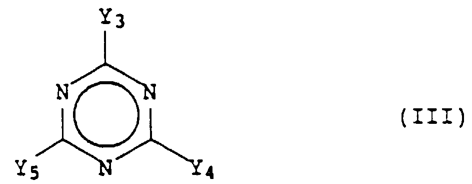 dans laquelle Y₃, Y₄ et Y₅, indépendamment les uns des autres, représentent chacun un groupe alkyle, alcényle, aralkyle éventuellement substitués ou un groupe pipéridino, un groupe -N(Y₆) (Y₇), un groupe -OY₈ ou un groupe -SY₉ dans lesquels Y₆, Y₇, Y₈ et Y₉, indépendamment les uns des autres, représentent chacun un atome d'hydrogène ou un groupe alkyle ou aryle éventuellement substitués, à condition qu'au moins l'un de Y₃, Y₄ et Y₉ représente un groupe alkyle mono-, di- ou trihalogéné, ou la 2-phényl-4,6-bis(trichlorométhyl)-s-triazine, la 2-(p-acétylphényl)-4,6-bis(trichlorométhyl)-s-triazine ou la 2-(p-chlorophényl)-4,6-bis(trichlorométhyl)-s-triazine.

2. Composition de photoresist négatif selon la revendication 1, dans laquelle le rapport molaire du p-hydroxystyrène au styrène dans le copolymère est de 70/30 à 85/15.

3. Composition de photoresist négatif selon la revendication 1, dans laquelle le poids moléculaire moyen en poids du copolymère est de 1 000 à 5 000.

4. Composition de photoresist négatif selon la revendication 1, dans laquelle le poids moléculaire moyen en poids du copolymère est de 2 500 à 4 000.

5. Composition de photoresist négatif selon la revendication 1, dans laquelle le groupe alkyle inférieur représenté par Rₐ est choisi dans le groupe constitué par les groupes méthyle, éthyle, n-propyle et n-butyle.

6. Composition de photoresist négatif selon la revendication 1, dans laquelle l'agent de réticulation est la mélamine ou la benzoguanamine.

7. Composition de photoresist négatif selon la revendication 1, dans laquelle le groupe alkyle représenté par Y₃, Y₄ et Y₅ est un groupe alkyle à chaîne linéaire ou ramifiée ayant 1 à 7 atomes de carbone qui peut être substitué par un atome d'halogène.

8. Composition de photoresist négatif selon la revendication 1, dans laquelle le groupe alkyle représenté par Y₃, Y₄ et Y₅ est choisi dans le groupe constitué par les groupes méthyle, éthyle, n-propyle, i-propyle, n-butyle et t-butyle qui peuvent être substitués par un atome d'halogène.

9. Composition de photoresist négatif selon la revendication 1, dans laquelle le groupe alcényle représenté par Y₃, Y₄ et Y₅ est un groupe alcényle à chaîne linéaire ou ramifiée ayant 2 à 7 atomes de carbone qui peut être substitué par un groupe aryle ou un groupe hétérocyclique.

10. Composition de photoresist négatif selon la revendication 1, dans laquelle le groupe aralkyle représenté par Y₃, Y₄ et Y₅ est un groupe phénylalkyle ayant 7 à 10 atomes de carbone.

11. Composition de photorésist négatif selon la revendication 1, dans laquelle le groupe alkyle éventuellement substitué représenté par Y₆ à Y₉ est un groupe alkyle à chaîne linéaire ou ramifiée ayant 1 à 6 atomes de carbone.

12. Composition de photorésist négatif selon la revendication 11, dans laquelle le groupe alkyle -éventuellement substitué représenté par Y₆ à Y₉ est choisi dans le groupe constitué par les groupes méthyle, éthyle, n-propyle et n-butyle.

13. Composition de photoresist négatif selon la revendication 1, dans laquelle le groupe aryle éventuellement substitué représenté par Y₆ à Y₉ est un groupe phényle ou naphtyle.

14. Composition de photoresist négatif selon la revendication 1, dans laquelle la composition comprend 40 à 80 % en poids du copolymère, 5 à 20 % en poids de l'agent de réticulation et 1 à 20 % en poids du précurseur d'acide photo-induit.
